Europäisches Patentamt

European Patent Office

Office européen des brevets

(11) Numéro de publication: **0 305 238 B1**

(12) **FASCICULE DE BREVET EUROPEEN**

(45) Date de publication de fascicule du brevet: **17.06.92**  (51) Int. Cl.⁵: **A61B  3/10**

(21) Numéro de dépôt: **88401942.3**

(22) Date de dépôt: **26.07.88**

(54) **Pupillomètre automatique.**

(30) Priorité: **30.07.87 FR 8710804**

(43) Date de publication de la demande:
**01.03.89 Bulletin  89/09**

(45) Mention de la délivrance du brevet:
**17.06.92 Bulletin  92/25**

(84) Etats contractants désignés:
**DE ES GB IT**

(56) Documents cités:
**EP-A- 0 115 723**

(73) Titulaire: **ESSILOR INTERNATIONAL, Cie Générale d'Optique**
**1 Rue Thomas Edison, Echat 902**
**F-94028 Créteil Cédex(FR)**

(72) Inventeur: **Bovet, Christian**
**196 Avenue de Versailles**
**F-75016 Paris(FR)**

(74) Mandataire: **CABINET BONNET-THIRION**
**95 Boulevard Beaumarchais**
**F-75003 Paris(FR)**

Rank Xerox (UK) Business Services

## Description

La présente invention concerne d'une manière générale la mesure d'un distance liée à l'écart pupillaire d'un individu, soit qu'il s'agisse, par exemple, directement, de cet écart pupillaire, soit qu'il s'agisse du demi-écart pupillaire correspondant.

A ce jour cette mesure se fait manuellement, par exemple à l'aide d'un appareil nommé pupillomètre.

Un tel pupillomètre se trouve notamment décrit dans le brevet français No 1.506.352.

Globalement, il comporte une source lumineuse propre à la génération d'un reflet cornéen sur l'un au moins des yeux de l'individu concerné, et, en pratique, sur chacun de ceux-ci, et un référentiel de mesure par rapport auquel est à situer un tel reflet cornéen.

En pratique, il est prévu, pour ce faire, deux repères mobiles, qui sont à faire coïncider chacun respectivement avec les deux reflets cornéens de l'individu, et dont la distance relative est ensuite à relever à l'aide du référentiel de mesure associé.

Bien que cette disposition, relativement rudimentaire, et donc, bon marché, ait donné et puisse encore donner satisfaction, elle présente notamment l'inconvénient de nécessiter le déplacement de réticules, et donc d'être relativement lente, et, quant à ses résultats, d'être sujette à l'habileté de l'opérateur.

La présente invention a d'une manière générale pour objet une disposition propre à permettre, de manière simple, la mesure automatique, et donc rapide, d'une distance liée à l'écart pupillaire d'un individu.

De manière plus précise, elle a tout d'abord pour objet un procédé, du genre consistant à mettre en oeuvre une source lumineuse pour la génération d'un reflet cornéen sur l'un au moins des yeux de l'individu concerné, et à situer, par rapport à un référentiel de mesure, le reflet cornéen ainsi produit, ce procédé étant d'une manière générale caractérisé en ce qu'on assure le balayage d'une transversale à la direction générale d'observation d'un individu, de manière à diriger sur un récepteur de détection dans lequel intervient le référentiel de mesure les rayons lumineux correspondant au(x) reflet(s) cornéen(s) à détecter ; elle a encore pour objet un pupillomètre mettant en oeuvre un tel procédé.

En pratique, le balayage suivant l'invention est assuré par l'image d'une fente donnant accès au récepteur de détection.

Quoi qu'il en soit, ce balayage permet de relever automatiquement et très simplement la présence du ou des reflets cornéens à détecter, et il fournit ainsi en vraie grandeur, quelle que soit la distance à laquelle se situe l'individu dans des limites déterminées par rapport à un plan moyen qui est le plan théorique des yeux, l'information d'écart pupillaire recherchée pour celui-ci.

De préférence, suivant un développement de l'invention, on forme le référentiel de mesure à l'aide d'une batterie de sources lumineuses de référence disposées linéairement suivant un pas régulier, et, en pratique, ces sources lumineuses de référence étant alignées suivant une transversale à la direction générale d'observation de l'individu, on forme ce référentiel de mesure par l'image de ces sources lumineuses de référence dans le récepteur de détection, en faisant suivre aux rayons lumineux correspondants, sur une partie au moins de leur cheminement optique, la même voie que celle suivie par les rayons lumineux correspondants aux reflets cornéens à détecter.

Ainsi se trouve systématiquement écartée, de manière avantageusement fiable et économique, l'incidence, sur le résultat obtenu, des inévitables variations de caractéristiques du balayage mis en oeuvre et/ou de l'oculaire par ailleurs également présent pour la formation des images requises, sans qu'il soit nécessaire de réaliser de façon précise, et donc coûteuse, ce balayage, et/ou cet oculaire.

En bref, la disposition suivant l'invention permet d'associer à une mise en oeuvre aisée et rapide l'avantage d'une réalisation bon marché.

Le pupillomètre correspondant est en outre avantageusement peu encombrant, et, ne mettant en oeuvre que des sources lumineuses ponctuelles qui ne sont chacune allumées qu'un court instant tout en autorisant de très fortes luminances, il est également très performant en énergie lumineuse et très économique d'emploi.

Les caractéristiques et avantages de l'invention ressortiront d'ailleurs de la description qui va suivre, à titre d'exemple, en référence aux dessins schématiques annexés sur lesquels :

la figure 1 est une vue en élévation coupe d'un pupillomètre suivant l'invention, suivant la ligne I-I de la figure 2 ;

la figure 2 en est une vue en plan, suivant la ligne II-II de la figure 1 ;

la figure 3 reprend, à échelle supérieure, le détail de la figure 1 repéré par un encart III sur cette figure 1 ;

la figure 4 est, à échelle supérieure, une vue en coupe longitudinale, suivant la ligne IV-IV de la figure 3, du générateur de référentiel de mesure partiellement représenté sur cette figure 3 ;

la figure 5 reprend, à l'échelle de la figure 3, le détail de la figure 1 repéré par un encart V sur cette figure 1 ;

la figure 6 est, à l'échelle de la figure 4, une vue en coupe longitudinale, suivant la ligne VI-VI de la figure 5, du générateur de reflet cornéen partiellement représenté sur cette figure 5 ;

la figure 7 est un diagramme illustrant dans son ensemble le mode d'intervention du pupillomètre suivant l'invention ;

les figures 8A, 8B sont des diagrammes donnant l'allure des signaux délivrés par le récepteur de détection que comporte ce pupillomètre.

Sur ces figures, on a schématisé en OD, OG les yeux de l'individu dont l'écart pupillaire global E ou les demi-écarts pupillaires ED, EG sont à relever, ces derniers étant appréciés par rapport à son axe nasal considéré ci-après comme définissant sa direction générale d'observation D.

De manière connue en soi, le pupillomètre 10 mis en oeuvre, suivant l'invention, pour le relevé correspondant, comporte, dans un bâti 11 en forme générale de caisson globalement parallélépipédique, un générateur de reflet cornéen 12, c'est-à-dire une source lumineuse propre à la génération d'un reflet cornéen sur l'un au moins des yeux OD, OG de l'individu concerné.

Dans la forme de réalisation représentée, ce générateur de reflet cornéen 12 intervient à travers une simple vitre 13, qui, commune par exemple aux deux yeux OD, OG, ferme, en façade, le pupillomètre 10, en étant légèrement inclinée sur la direction générale d'observation D de l'individu.

En pratique, cette direction générale d'observation D se confond avec l'axe optique de l'appareil.

Pour une meilleure assise de l'individu, le bâti 11 est doté d'un appui contre lequel il doit venir porter.

Dans la forme de réalisation représentée, il s'agit d'un appui nasal 14, mais, en variante, il pourrait tout aussi bien s'agir d'un appui frontal.

En pratique, le générateur de reflet cornéen 12 est disposé à l'écart de la direction générale d'observation D de l'individu, sous cette direction générale d'observation D, à la partie basse du bâti 11, et au voisinage de la vitre 13.

Dans la forme de réalisation représentée, il comporte deux sources lumineuses 12D, 12G propres chacune respectivement à la génération d'un reflet cornéen, à raison d'une par oeil, la source lumineuse 12D pour l'oeil droit OD et la source lumineuse 12G pour l'oeil gauche OG.

En pratique, chacune de ces sources lumineuses 12D, 12G comporte une pluralité de sources lumineuses élémentaires 16, qui, au nombre de quatre dans la forme de réalisation représentée, sont alignées, à un pas régulier, suivant une transversale, qui leur est commune, à la direction générale ation D de l'individu concerné.

Dans la forme de réalisation représentée, ces sources lumineuses élémentaires 16 sont constituées par des ouvertures rectangulaires de largeur définie ménagées dans la paroi de façade d'un caisson, respectivement 17D, 17G, dans lequel interviennent, en nombre égal, et suivant le même pas, des diodes lumineuses 18.

De manière connue en soi, et suivant des modalités décrites plus en détail ultérieurement, le pupillomètre 10 suivant l'invention comporte en outre un référentiel de mesure, qui est non visible en soi sur les figures, et par rapport auquel sont à situer les reflets cornéens formés sur l'oeil droit OD et sur l'oeil gauche OG de l'individu concerné.

Suivant l'invention, le pupillomètre 10 comporte un récepteur de détection 20, dans lequel intervient le référentiel de mesure, et un dispositif de balayage 21, qui est propre à assurer le balayage d'une transversale à la direction générale d'observation D de l'individu, de manière à diriger sur le récepteur de détection 20 les rayons lumineux correspondant aux reflets cornéens à détecter.

En pratique, ce balayage est fait par l'image d'une fente 22 donnant accès au récepteur de détection 20.

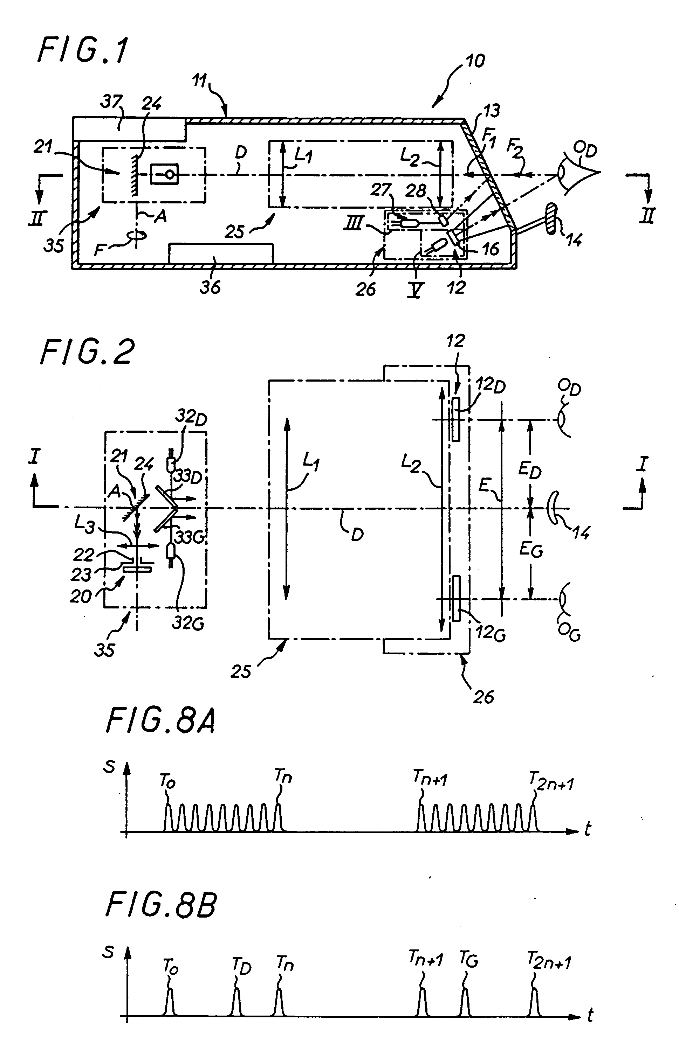

En pratique, également, le récepteur de détection 20 est disposé latéralement par rapport à la direction générale d'observation D de l'individu, avec, en avant de lui, un masque 23 comportant le fente 22 contrôlant son accès, et le dispositif de balayage 21 comporte un miroir tournant 24, qui, interposé sur ladite direction générale d'observation D, au droit dudit récepteur de détection 20, et tel que schématisé par une flèche F sur la figure 1, est monté rotatif autour d'un axe A perpendiculaire au plan que forment conjointement cette direction générale d'observation D et ce récepteur de détection 20.

Un oculaire 25, formée de deux lentilles L1, L2 dans la forme de réalisation représentée, étant interposé sur la direction générale d'observation D de l'individu, le miroir tournant 24 est placé au foyer objet de cet oculaire 25, au moins lorsque la mesure à effectuer doit correspondre à la vision de loin de cet individu, tandis que, sous le contrôle de l'appui nasal 14, le plan théorique des yeux OD, OG de celui-ci est situé à son foyer image.

Conjointement, une lentille L3 interposée entre le masque 23 et le miroir tournant 24 forme à l'infini une image de la fente 22 de ce masque 23.

Il résulte de ce qui précède que l'oculaire 25 reforme l'image de cette fente 22 dans le plan théorique des yeux de l'individu concerné, et que par rotation du miroir tournant 24, on obtient un balayage par l'image de cette fente 22 d'une transversale à la direction générale d'observation D de celui-ci.

En pratique, le générateur de reflet cornéen 12 appartient à un émetteur 26 comportant également, pour la formation du référentiel de mesure, au moins une batterie 27 de sources lumineuses de référence 28, qui sont disposées linéairement, suivant un pas régulier, le long d'une transversale à la direction générale d'observation D de l'individu, et qui, comme les reflets cornéens à détecter, sont soumises au dispositif de balayage 21.

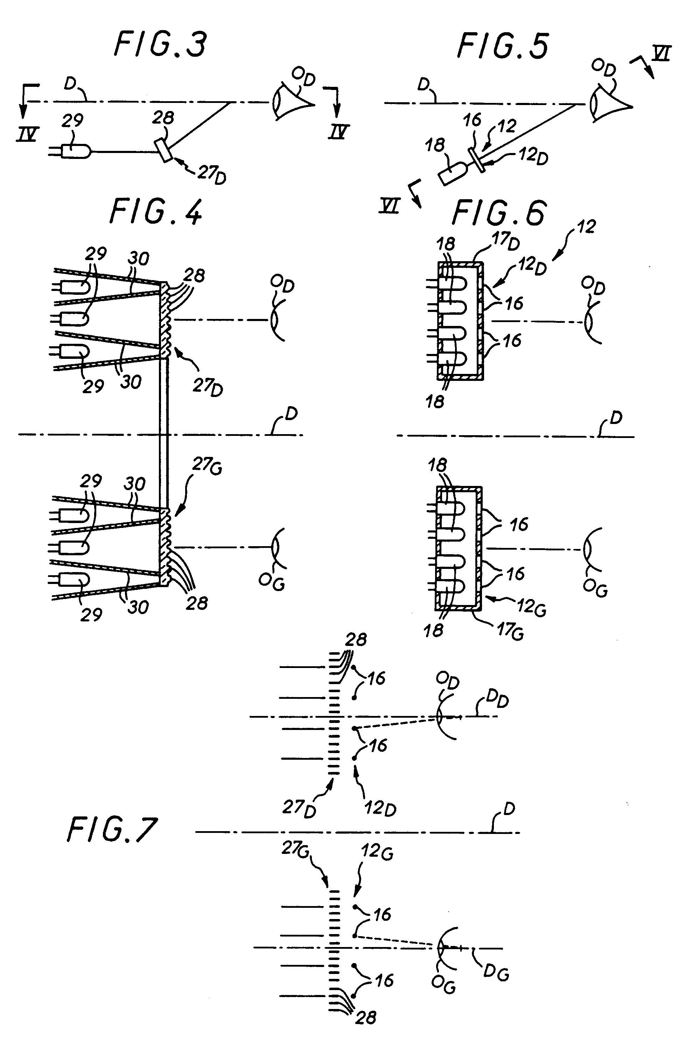

Dans la forme de réalisation représentée, il y a, disposées chacune respectivement de part et d'autre de la direction générale d'observation D de l'individu, en dessous de celle-ci, deux batteries 27D, 27G de sources lumineuses de référence 28, à raison d'une par oeil, la batterie 27D pour l'oeil droit OD, et la batterie 27G pour l'oeil gauche OG, figure 4, et il intervient donc, dans ce cas, pour un même récepteur de détection 20, deux référentiels de mesure élémentaires, à raison d'un par oeil.

Chacune de ces batteries 27D, 27G de sources lumineuses de référence 28 est constituée, dans cette forme de réalisation, par un réseau de micro-lentilles cylindriques parallèles.

Il s'agit, globalement, en l'espèce, de nervures parallèles de contour transversal hémi-circulaire présentes en saillie, à raison d'une nervure par source lumineuse de référence 28 désirée, sur la face avant d'une plaque transparente, qui peut être commune à l'une et l'autre des batteries 27D, 27G concernées, et qui peut par exemple être obtenue par montage d'une quelconque matière synthétique.

En arrière des micro-lentilles cylindriques parallèles ainsi constituées se trouve disposée au moins une source lumineuse proprement dite, en l'espèce une diode lumineuse.

En pratique, dans la forme de réalisation représentée, il y a ainsi trois diodes lumineuses 29 pour chacune des batteries 27D, 27G de sources lumineuses de référence 28, à savoir une diode lumineuse 29 pour chacune des sources lumineuses de référence 28 disposées aux extrémités d'une telle batterie, et une diode lumineuse 29 pour l'ensemble des autres sources lumineuses de référence 28 de celle-ci, avec, séparant l'une de l'autre les diodes lumineuses 29 ainsi mises en oeuvre, des écrans 30 s'étendant jusqu'au réseau de micro-lentilles correspondant.

De préférence, et tel que représenté, sur une partie au moins de leur cheminement optique, les rayons lumineux, schématisés par une simple flèche F1 sur la figure 1, correspondant aux sources lumineuses de référence 28 suivent la même voie que celle suivie par les rayons lumineux, schématisés par la double flèche F2, correspondant aux reflets cornéens à détecter.

En pratique, il en est ainsi tout au long de la direction générale d'observation D de l'individu à compter de la vitre 13, et jusqu'au récepteur de détection 20.

En effet, appartenant, comme le générateur de reflet cornéen 12, à l'émetteur 26, les sources lumineuses de référence 28 sont disposées au voisinage de la vitre 13, au-dessus dudit générateur de reflet cornéen 12, et cette vitre 13 forme pour elles, ou, plus exactement, pour les rayons lumineux qui en sont issus, un miroir réfléchissant.

Dès lors, comme les rayons lumineux correspondant aux reflets cornéens à détecter, les rayons lumineux correspondant aux sources lumineuses de référence 28 traversent, suivant la direction générale d'observation D de l'individu, l'oculaire 25, avant d'être réfléchis, en direction du récepteur de détection 20, par le miroir tournant 24 du dispositif de balayage 21.

Pour la fixation du regard de l'individu, le pupillomètre 10 suivant l'invention comporte, en outre, au moins une source lumineuse supplémentaire dont il est donné audit individu une image lui apparaissant à l'infini lorsque la mesure à effectuer doit correspondre à sa vision de loin.

En pratique, dans la forme de réalisation représentée, deux sources lumineuses supplémentaires 32D, 32G sont ainsi prévues, à raison d'une par oeil, la source lumineuse supplémentaire 32D pour l'oeil droit OD et la source lumineuse supplémentaire 32G pour l'oeil gauche OG, et leurs images ne sont chacune respectivement visibles que de l'oeil correspondant.

A cet effet, ces sources lumineuses supplémentaires 32D, 32G sont disposées chacune respectivement de part et d'autre de la direction générale d'observation D de l'individu, en regard l'une de l'autre, entre l'oculaire 25 et le miroir tournant 24 du dispositif de balayage 21, et elles interviennent chacune respectivement par l'intermédiaire d'une lame mince et claire 33D, 33G qui en fournit une image située au foyer objet de cet oculaire 25 lorsque la mesure à effectuer doit correspondre à la vision de loin.

Comme précédemment, ces sources lumineuses supplémentaires 32D, 32G peuvent être chacune constituées par une diode émettrice, et, par exemple, une diode verte.

Le récepteur de détection 20 est, en pratique, un récepteur photosensible, ou, autrement dit, une photo diode.

De préférence, et tel que schématisé sur les figures, il appartient, avec le dispositif de balayage 21, à un sous-ensemble 35, qui, pour permettre, si désiré, non plus seulement une mesure en vision de loin, mais également une mesure en vision de près, est monté mobile le long de la direction d'observation D de l'individu.

En effet, prise dans son ensemble, l'imagerie de la fente 22 du masque 23 n'est pas affectée par le déplacement d'un tel sous-ensemble 35.

Corollairement, l'oculaire 25 peut avantageusement être fixe. De préférence, et tel que représenté, les sources lumineuses supplémentaires 32D, 32G mises en oeuvre pour la fixation du regard de l'individu appartiennent, elles aussi, avec les lames minces et claires 33D, 33G qui leur sont associées, au sous-ensemble mobile 35.

Suivant des modalités qui, relevant de l'homme de l'art, et n'appartenant pas par elles-mêmes à la présente invention, ne seront pas décrites en détail ici, le pupillomètre 10 suivant l'invention se complète par des moyens d'alimentation propres à l'excitation des diverses diodes mises en oeuvre, aussi bien qu'à celle du moteur prévu par ailleurs pour la commande en rotation du miroir tournant 24 du dispositif de balayage 21, et, tel que schématisé à la figure 1, par un dispositif de commande 36, en pratique électronique, propre à un contrôle de l'ensemble, et par des moyens d'affichage 37 propres à une visualisation des résultats obtenus.

En service, et tel que schématisé par des tirets sur la figure 7, chacune des micro-lentilles cylindriques constituant les sources lumineuses de référence 28 donne de la diode lumineuse 29 qui lui est attribuée une image ayant la forme d'un segment lumineux.

Autrement dit, en service, les sources lumineuses de référence 28 se présentent sous la forme de segments lumineux parallèles.

Par contre, chacune des sources lumineuses élémentaires 16 que comporte le générateur de reflet cornéen 12 se présente, conjointement, sous la forme d'un point lumineux, à l'image de l'ouverture qui la constitue.

En service, chacune de ces sources lumineuses élémentaires 16 crée à tour de rôle un reflet cornéen.

Du fait du dispositif de balayage 21, ou, plus exactement, du fait de la synchronisation entre le balayage assuré par celui-ci et l'allumage des sources lumineuses élémentaires 16, le reflet cornéen traité par le récepteur de détection 20 est celui créé par la source lumineuse élémentaire 16 la plus proche de la direction particulière d'observation DD, DG de l'oeil OD, OG correspondant, figure 7.

Pour mesurer, à l'aide d'un tableau de conversion, les demi-écarts pupillaires ED, EG par rapport à la direction générale d'observation D de l'individu, il suffit de situer, par rapport aux sources lumineuses de référence 28, et suivant les directions particulières d'observation DD, DG correspondantes, qui sont parallèles à la direction générale d'observation D, les reflets cornéens ainsi formés.

L'écart pupillaire E est la somme de ces demi-écarts pupillaires ED, EG.

En pratique, par l'oculaire 25 et le dispositif de balayage 21, le relevé correspondant est effectuée dans le récepteur de détection 20.

C'est la raison pour laquelle, par transposition, le référentiel de mesure mis en oeuvre est constitué par l'image, dans ce récepteur de détection 20, des sources lumineuses de référence 28.

En pratique, et tel que schématisés par les diagrammes des figures 8A et 8B, sur lesquels il est porté, en abscisses, le temps t, et en ordonnées le signal S délivré par le récepteur de détection 20, il est procédé en deux phases.

Dans une première phase, figure 8A, on ne fait intervenir, dans le récepteur de détection 20, que le seul référentiel de mesure.

Autrement dit, dans cette première phase, seules les sources lumineuses de référence 28 sont opératives, seules étant éclairées les diodes lumineuses 29 dont elles sont déduites.

Compte tenu du balayage assuré par le miroir tournant 24, le signal délivré par le récepteur de détection 20 se présente donc sous la forme d'une succession de pics T, à raison d'un par source lumineuse de référence 28.

Soit $T_0$ à $T_n$ les pics correspondant à la batterie 27D de sources lumineuses de référence 28, et donc à l'oeil droit OD de l'individu, et soit $T_n + 1$ à $T_{2n} + 1$, ceux correspondant à la batterie 27G de sources lumineuses de référence 28, et donc à l'oeil gauche OG de cet individu.

Les valeurs correspondant à ces pics $T_0$ à $T_{2n} + 1$ sont mémorisées.

Cette première phase permet donc de relever, en quelque sort, la géométrie d'ensemble du référentiel de mesure mis en oeuvre, et, de la première phase à la deuxième, il intervient une mise en mémoire du référentiel de mesure ainsi formé et relevé.

Dans la deuxième phase, et toujours sous les effets du balayage assuré par le miroir tournant 24, on superpose, dans le récepteur de détection 20, une fraction seulement du référentiel de mesure et les reflets cornéens à détecter.

En pratique, au cours de cette deuxième phase, qui permet de réaliser la mesure proprement dite, seules les extrémités du référentiel de mesure sont retenues.

Autrement dit, seules sont alors opératives les sources lumineuses de référence 28 disposées aux extrémités des batteries 27D, 27G, seules étant excitées les diodes lumineuses 29 correspondantes.

Ainsi, le signal délivré par le récepteur de détection 20 ne comporte plus, pour le référentiel de mesure, que les seuls pics $T_0$ et $T_n$, d'une part, et $T_n + 1$ et $T_{2n} + 1$, d'autre part, correspondant aux sources lumineuses de référence 28 extrêmes.

Mais, conjointement, le générateur de reflet cornéen 12 étant actif, il apparaît, entre ces pics, des pics TD, TG correspondant chacun respectivement au reflet cornéen dû à l'oeil droit OD de l'individu et à celui dû à son oeil gauche OG.

Un calcul par règle de trois permet de connaître les positions de ces pics TD, TG par rapport aux pics To à $T_{2n}$ + 1 précédemment mémorisés.

Ainsi qu'il est aisé de le comprendre, toute éventuelle irrégularité dans la géométrie du référentiel de mesure, due par exemple à une fluctuation cyclique de la vitesse de rotation du moteur assurant l'entraînement du miroir tournant 24, se trouve n'avoir de ce fait aucune incidence sur la précision de la mesure effectuée.

Par exemple, et préférentiellement, le miroir tournant 24 tourne toujours dans le même sens.

Mais, en variante, il peut tourner dans un sens pour la première phase, celle assurant le relevé de la géométrie du référentiel de mesure, et dans le sens opposé pour la deuxième phase, celle au cours de laquelle on situe par rapport à ce référentiel de mesure les reflets cornéens à détecter.

Quoi qu'il en soit, le fait de ne retenir, au cours de cette deuxième phase, que les sources lumineuses de référence extrêmes, permet d'éviter que ces reflets cornéens soient occultés par une quelconque des sources lumineuses de références intermédiaires.

Quoi qu'il en soit, également, les diverses sources lumineuses mises en oeuvre doivent évidemment être préférentiellement allumées en synchronisme avec le balayage effectué.

Il est donc souhaitable, pour ce faire, que les éventuelles variations de la vitesse de rotation moyenne du miroir tournant 24 entre les deux phases restent faibles.

La précision du pupillomètre 10 suivant l'invention dépend, notamment, de la géométrie des micro-lentilles cylindriques mises en oeuvre pour la constitution du référentiel de mesure.

Mais, en pratique, une précision de ± 0,25 minutes d'angle suffit pour le montage correct de verres sur une monture de lunettes.

L'incidence de la géométrie de ces micro-lentilles cylindriques sur la précision du pupillomètre 10 suivant l'invention n'est donc pas déterminante.

Quant à sa sensibilité, elle dépend, notamment, de la largeur de la fente 22 donnant accès à son récepteur de détection 20.

Mais celle-ci n'est pas non plus critique.

La présente invention ne se limite d'ailleurs pas à la forme de réalisation décrite et représentée, mais englobe toute variante d'exécution.

En outre, au lieu de la mesure directe de l'écart pupillaire de l'individu concerné, il peut être procédé à la mesure d'une quelconque distance simplement liée de manière connue à cet écart pupillaire.

## Revendications

1. Procédé pour la mesure automatique d'une distance liée à l'écart pupillaire (E) d'un individu, du genre consistant à mettre en oeuvre une source lumineuse (12) pour la génération d'un reflet cornéen sur l'un au moins des yeux de cet individu, et à situer, par rapport à un référentiel de mesure, le reflet cornéen ainsi produit, caractérisé en ce qu'on assure le balayage d'une transversale à la direction générale d'observation (D) de l'individu, de manière à diriger sur un récepteur de détection (20) dans lequel intervient le référentiel de mesure, les rayons lumineux correspondant au(x) reflet(s) cornéen(s) à détecter.

2. Procédé suivant la revendication 1, caractérisé en ce qu'on assure le balayage d'une transversale à la direction générale d'observation (D) de l'individu par l'image d'une fente (22) donnant accès au récepteur de détection (20).

3. Procédé suivant l'une quelconque des revendications 1, 2, caractérisé en ce qu'on forme le référentiel de mesure à l'aide d'une batterie de sources lumineuses de référence (28) disposées linéairement suivant un pas régulier.

4. Procédé suivant la revendication 3, caractérisé en ce que, les sources lumineuses de référence (28) étant alignées suivant une transversale à la direction générale d'observation (D) de l'individu, on forme le référentiel de mesure par l'image de ces sources lumineuses de référence (28) dans le récepteur de détection (20), en faisant suivre aux rayons lumineux correspondants, sur une partie au moins de leur cheminement optique, la même voie que celle suivie par les rayons lumineux correspondant au reflet cornéen à détecter.

5. Procédé suivant l'une quelconque des revendications 3, 4, caractérisé en ce que, dans une première phase, on ne fait intervenir dans le récepteur de détection (20) que le seul référentiel de mesure, puis, dans une deuxième phase, on y superpose une fraction seulement de ce référentiel de mesure, éventuellement limitée à ses seules extrémités, et le reflet cornéen à détecter.

6. Procédé suivant la revendication 5, caractérisé en ce que, de la première phase à la deuxième, intervient une mise en mémoire du référentiel de mesure.

7. Procédé suivant l'une quelconque des revendications 1 à 6, caractérisé en ce que, pour un même récepteur de détection (20), on fait intervenir, comme référentiel de mesure, deux référentiels de mesure élémentaires, à raison d'un par oeil.

8. Pupillomètre, du genre mettant en oeuvre une source lumineuse propre à la génération d'un reflet cornéen sur l'un au moins des yeux d'un individu, et un référentiel de mesure, par rapport auquel ce reflet cornéen est à situer, caractérisé en ce qu'il comporte un récepteur de détection (20) dans lequel intervient le référentiel de mesure, et un dispositif de balayage (21), qui est propre à assurer le balayage d'une transversale à la direction générale d'observation (D) de l'individu concerné, de manière à diriger sur le récepteur de détection (20) les rayons lumineux correspondant au(x) reflet(s) cornéen(s) à détecter.

9. Pupillomètre suivant la revendication 8, caractérisé en ce que le dispositif de balayage mis en oeuvre est propre à assurer le balayage d'une transversale à la direction générale d'observation (D) de l'individu par l'image d'une fente (22) donnant accès au récepteur de détection (20).

10. Pupillomètre suivant l'une quelconque des revendications 8, 9, caractérisé en ce que le récepteur de détection (20) est disposé latéralement par rapport à la direction générale d'observation (D) de l'individu, et le dispositif de balayage (21) comporte un miroir tournant (24), qui, interposé sur ladite direction générale d'observation (D), au droit dudit récepteur de détection (20), est monté rotatif autour d'un axe (A) perpendiculaire au plan que forment conjointement cette direction générale d'observation (D) et ce récepteur de détection (20).

11. Pupillomètre suivant la revendication 10, caractérisé en ce que, un oculaire (25) étant interposé sur la direction générale d'observation (D) de l'individu, le miroir tournant (24) est placé au foyer objet dudit oculaire (25) lorsque la mesure à effectuer correspond à la vision de loin de celui-ci.

12. Pupillomètre suivant la revendication 11, caractérisé en ce que l'oculaire est fixe.

13. Pupillomètre suivant l'une quelconque des revendications 8 à 12, caractérisé en ce que le récepteur de détection (20) et le dispositif de balayage (21) appartiennent à un sous-ensemble (35) monté mobile le long de la direction générale d'observation (D) de l'individu.

14. Pupillomètre suivant l'une quelconque des revendications 8 à 13, caractérisé en ce que, pour le fixation du regard de l'individu, il comporte au moins une source lumineuse supplémentaire (32D, 32G) dont il est donné audit individu une image lui apparaissant à l'infini.

15. Pupillomètre suivant les revendications 13 et 14, prises conjointement, caractérisé en ce que ladite source lumineuse supplémentaire (32D, 32G) appartient au sous-ensemble mobile (35).

16. Pupillomètre suivant l'une quelconque des revendications 14, 15, caractérisé en ce qu'il y a deux sources lumineuses supplémentaires (32D, 32G), une par oeil, et leurs images ne sont chacune respectivement visibles que de l'oeil correspondant.

17. Pupillomètre suivant l'une quelconque des revendications 8 à 16, caractérisé en ce que la source lumineuse (12) propre à la génération d'un reflet cornéen intervient, soit directement, soit à travers une simple vitre (13).

18. Pupillomètre suivant l'une quelconque des revendications 8 à 17, caractérisé en ce que la source lumineuse (12) propre à la génération d'un reflet cornéen comporte une pluralité de sources lumineuses élémentaires (16), alignées suivant une transversale à la direction générale d'observation (D) de l'individu.

19. Pupillomètre suivant l'une quelconque des revendications 8 à 18, caractérisé en ce qu'il y a deux sources lumineuses (12D, 12G) propres à la génération d'un reflet cornéen, à raison d'une par oeil.

20. Pupillomètre suivant l'une quelconque des revendications 8 à 19, caractérisé en ce que, pour la formation du référentiel de mesure, il comporte au moins une batterie de sources lumineuses de référence (28), qui sont disposées linéairement suivant un pas régulier le long d'une transversale à la direction générale d'observation (D) de l'individu, et qui, comme le reflet cornéen à détecter, sont soumises au dispositif de balayage (21).

21. Pupillomètre suivant la revendication 20, caractérisé en ce que, sur une partie au moins de leur cheminement optique, les rayons lumineux correspondant auxdites sources lumineuses de référence (28) suivent la même voie que celle suivie par les rayons lumineux correspondant au(x) reflet(s) cornéen(s) à détecter.

22. Pupillomètre suivant la revendication 21, caractérisé en ce que, une vitre (13) étant prévue à travers laquelle intervient la source lumineuse (12) propre à la génération d'un reflet cornéen, lesdites sources lumineuses de référence (28) sont disposées au voisinage de ladite vitre (13) et celle-ci forme un miroir réfléchissant pour les rayons lumineux qui en sont issus.

23. Pupillomètre suivant l'une quelconque des revendications 20 à 22, caractérisé en ce que la batterie de sources lumineuses de référence (28) est formée par un réseau de micro-lentilles cylindriques parallèles en arrière duquel est disposée au moins une source lumineuse proprement dite (29).

24. Pupillomètre suivant l'une quelconque des revendications 20 à 23, caractérisé en ce qu'il y a deux batteries (27D, 27G) de sources lumineuses de référence (28), à raison d'une par oeil.

25. Pupillomètre suivant l'une quelconque des revendications 8 à 24, caractérisé en ce que le récepteur de détection (20) est un récepteur photo-sensible.

**Claims**

1. A process for automatically measuring a distance related to the interpupillary distance (E) of a person, of the type comprising using a light source (12) for generating a corneal reflection on one at least of the eyes of said person, and locating the corneal reflection produced in that way, with respect to a measurement reference, characterised by effecting scanning of a line transverse to the general direction of observation (D) of the person, in such a way as to direct the light rays corresponding to the corneal reflection or reflections to be detected, on to a detection receiver (20) in which the measurement reference occurs.

2. A process according to clad 1 characterised by effecting scanning of a line transverse to the general direction of observation (D) of the person by bans of the image of a slit (22) giving access to the detection receiver (20).

3. A process according to either one of clad 1 and 2 characterised by forming the measurement reference by means of a set of reference light sources (28) which are linearly disposed at regular spacings.

4. A process according to claim 3 characterised in that, the reference light sources (28) being aligned along a line transverse to the general direction of observation (D) of the person, the measurement reference is formed by bans of the image of said reference light sources (28) in the detection receiver (20), by causing the corresponding light rays, over a part at least of their optical path, to follow the sane path as that followed by the light rays corresponding to the corneal reflection to be detected.

5. A process according to either one of claims 3 and 4 characterised in that, in a first phase, only the measurement reference is caused to act in the detection receiver (20) and then, in a second phase, superimposed thereon is a fraction only of said measurement reference, possibly limited to just its ends, and the corneal reflection to be detected.

6. A process according to claim 5 characterised in that the measurement reference is memorised from the first phase to the second phase.

7. A process according to any one of claims 1 to 6 characterised in that two elementary measurement references, one for each eye, are used as a measurement reference, for a single detection receiver (20).

8. A pupillometer of the type using a light source for generating a corneal reflection on one at least of the eyes of a person, and a measurement reference, with respect to which said corneal reflection is to be located, characterised in that it comprises a detection receiver (20) in which the measurement reference occurs, and a scanning device (21) for effecting scanning of a line transverse to the general direction of observation (D) of the person in question, so as to direct on to the detection receiver (20) the light rays corresponding to the corneal reflection or reflections to be detected.

9. A papillometer according to claim 8 characterised in that the scanning device used is capable of effecting scanning of a line transverse to the general direction of observation (D) of the person by bans of the image of a slit (22) giving access to the detection receiver (20).

10. A pupillometer according to either one of claims 8 and 9 characterised in that the detection receiver (20) is disposed laterally with respect to the general direction of observation (D) of the person, and the scanning device (21) comprises a rotary r (24) which, interposed in said general direction of observation (D), at right angles to said detection receiver (20), is mounted rotatably about an axis (A) perpendicular to the plane jointly formed by said general direction of observation (D) and said detection receiver (20).

11. A pupillometer according to claim 10 characterised in that, an eyepiece (25) being interposed in the general direction of observation (D) of the person, the rotary mirror (24) is disposed at the object focus of the eyepiece (25) when the measurement to be effected corresponds to the far vision thereof.

12. A pepillometer according to claim 11 characterised in that the eyepiece is fixed.

13. A pupillometer according to any one of claims 8 to 12 characterised in that the detection receiver (20) and the scanning device (21) belong to a sub-assembly (35) which is mounted movably along the general direction of observation (D) of the person.

14. A pupillometer according to any one of claims 8 to 13 characterised in that, for fixing the gaze of the person, it comprises at least one supplementary light source (32D, 32G), an image thereof which appears at infinity to the person being given to the person.

15. A pupillometer according to claims 13 and 14 in combination characterised in that said supplementary light source (32D, 32G) belongs to the movable sub-assembly (35).

16. A pupillometer according to either one of claims 14 and 15 characterised in that there are two supplementary light sources (32D, 32G), one per eye, and their images are each respectively visible only by the corresponding eye.

17. A pupillometer according to any one of claims 8 to 16 characterised in that the light source (12) for the generation of a corneal reflection is involved either directly or through a simple window (13).

18. A pupillometer according to any one of claims 8 to 17 characterised in that the light source (12) for the generation of a corneal reflection comprises a plurality of elementary light sources (16) which are aligned along a line transverse to the general direction of observation (D) of the person.

19. A pupillometer according to any one of claims 8 to 18 characterised in that there are two light sources (12D, 12G) for the generation of a corneal reflection, one per eye.

20. A pupillometer according to any one of claims 8 to 19 characterised in that, for formation of the measurement reference, it comprises at least one set of reference light sources (28) which are disposed linearly at regular spacings along a line transverse to the general direction of observation (D) of the person and which, like the corneal reflection to be detected, are subjected to the scanning device (21).

21. A pupillometer according to claim 20 characterised in that, over a part at least of their optical path, the light rays corresponding to said reference light sources (28) follow the sane path as that follow by the light rays corresponding to the corneal reflection or reflections to be detected.

22. A pupillometer according to clam 21 characterised In that, a window (13) being provided through which the light source (12) for the generation of a corneal reflection is operable, said reference light sources (28) are disposed in the vicinity of said window (13) and the latter forms a reflecting mirror for the light rays which issue therefrom.

23. A pupillometer according to any one of claims 20 to 22 characterised in that the set of reference light sources (28) is formed by a system of parallel cylindrical microlenses, behind which is disposed at least one light source (29) In the true sense.

24. A pupillometer according to any one of claims 20 to 23 characterised in that there are two sets (27D, 27G) of reference light sources (28), one per eye.

25. A pupillometer according to any one of claims 8 to 24 characterised in that the detection receiver (20) is a photosensitive receiver.

**Patentansprüche**

1. Verfahren zur automatischen Messung eines Abstands, der verbunden ist mit dem Pupillenabstand (E) einer Person, derart, daß man eine Lichtquelle (12) zur Erzeugung eines Hornreflexes auf wenigstens einem der Augen der Person in Betrieb setzt, und den so hergestellten Hornreflex bezüglich eines Meß-Bezugssystems einstellt, <u>dadurch gekennzeichnet, daß</u>

man die Abtastung einer Transversalen zur allgemeinen Beobachtungsrichtung (D) der Person gewährleistet, derart, daß man die dem zu erfassenden Hornreflex entsprechenden Lichtstrahlen auf einen Erfassungsempfänger (20) richtet, in dem das Meß-Bezugssystem sich befindet.

2. Verfahren nach Anspruch 1, dadurch gekennzeichnet, daß man die Abtastung einer Transversalen zur allgemeinen Beobachtungsrichtung (D) der Person durch das Bild eines Spalts (22) gewährleistet, das Zugang zu dem Erfassungsempfänger (20) gibt.

3. Verfahren nach einem der Ansprüche 1, 2, dadurch gekennzeichnet, daß man das Meß-Bezugssystem mit Hilfe einer Gruppe von Bezugs-Lichtquellen (28) bildet, die linear gemäß einem regelmäßigen Abstand angeordnet sind.

4. Verfahren nach Anspruch 3, dadurch gekennzeichnet, daß die Bezugs-Lichtquellen (28) gemäß einer Transversalen zur allgemeinen Beobachtungsrichtung (D) der Person ausgerichtet sind, man das Meß-Bezugssystem durch das Bild der Bezugs-Lichtquellen (28) in dem Erfassungsempfänger (20) bildet, indem man den entsprechenden Lichtquellen auf wenigstens einem Teil ihres optischen Wegs denselben Weg folgen läßt, wie den, der durch die dem zu erfassenden Hornreflex entsprechenden Lichtstrahlen gefolgt wurde.

5. Verfahren nach einem der Ansprüche 3, 4, dadurch gekennzeichnet, daß man in einer ersten Phase in den Erfassungsempfänger (20) nur das Meß-Bezugssystem einläßt, dann in einer zweiten Phase man lediglich einen Bruchteil des Bezugs-Meßsystems ggf. beschränkt an seinen Enden und den zu erfassenden Hornreflex überlagert.

6. Verfahren nach Anspruch 5, dadurch gekennzeichnet, daß von der ersten Phase zur zweiten eine Speicherung des Meß-Bezugssystems erfolgt.

7. Vorrichtung nach einem der Ansprüche 1 bis 6, dadurch gekennzeichnet, daß man für einen gleichen Erfassungsempfänger (20) als Meß-Bezugssystem zwei elementare Meß-Bezugssysteme einsetzt, und zwar einen pro Auge.

8. Pupillometer, derart, der eine Lichtquelle in Betrieb nimmt, die zur Erzeugung eines Hornreflexes auf mindestens einem der Augen einer Person geeignet ist, und ein Meß-Bezugssystem, bezüglich dessen der Hornreflex gelegen ist, dadurch gekennzeichnet, daß er einen Erfassungsempfänger (20) aufweist, in dem das Meß-Bezugssystem auftritt, und eine Abtastvorrichtung (21), die geeignet ist, die Abtastung einer Transversalen zur allgemeinen Beobachtungsrichtung (D) der betroffenen Person zu gewährleisten, derart, daß man die dem zu erfassenden Hornreflex entsprechenden Lichtstrahlen auf den Erfassungsempfänger (20) lenkt.

9. Pupillometer nach Anspruch 8, dadurch gekennzeichnet, daß die eingesetzte Abtastvorrichtung geeignet ist, die Abtastung einer Transversalen zur allgemeinen Beobachtungsrichtung (D) der Person durch das Bild eines Spalts (22) zu gewährleisten, der einen Zugang zum Erfassungsempfänger (20) gibt.

10. Pupillometer nach einem der Ansprüche 8, 9, dadurch gekennzeichnet, daß der Erfassungsempfänger (20) quer bezüglich der allgemeinen Beobachtungsrichtung (D) der Person angeordnet ist, und die Abtastvorrichtung (21) einen Drehspiegel (24) aufweist, der auf der allgemeinen Beobachtungsrichtung (D) rechts von dem Erfassungsempfänger (20) eingefügt ist und um eine Achse (A) drehbar gelagert ist, die senkrecht zur Ebene steht, welche die allgemeine Beobachtungsrichtung (D) und der Erfassungsempfänger (20) zusammen bilden.

11. Pupillometer nach Anspruch 10, dadurch gekennzeichnet, daß ein Okular (25) in der allgemeinen Beobachtungsrichtung (D) der Person eingefügt ist, der Drehspiegel (24) im Brennpunkt des Okulars (25) angeordnet ist, wenn die durchzuführende Messung der Weitsicht dessen entspricht.

12. Pupillometer nach Anspruch 11, dadurch gekennzeichnet, daß das Okular fest ist.

13. Pupillometer nach einem der Ansprüche 8 bis 12, dadurch gekennzeichnet, daß der Erfassungsempfänger (20) und die Abtastvorrichtung (21) einer Untervorrichtung (35) angehören, die längs der allgemeinen Beobachtungsrichtung (D) der Person beweglich gelagert ist.

14. Pupillometer nach einem der Ansprüche 8 bis 13, dadurch gekennzeichnet, daß es für die Befestigung des Blicks der Person wenigstens eine ergänzende Lichtquelle (32D, 32G) aufweist, durch die der Person ein Bild gegeben ist, das ihr im Unendlichen erscheint.

**15.** Pupillometer nach den Ansprüchen 13 und 14, zusammengenommen, dadurch gekennzeichnet, daß die ergänzende Lichtquelle (32D, 32G) der beweglichen Untervorrichtung (35) angehört.

**16.** Pupillometer nach einem der Ansprüche 14, 15, dadurch gekennzeichnet, daß es zwei ergänzende Lichtquellen (32D, 32G) gibt, und zwar eine pro Auge, und ihre Bilder nur jeweils vom entsprechenden Auge sichtbar sind.

**17.** Pupillometer nach einem der Ansprüche 8 bis 16, dadurch gekennzeichnet, daß die Lichtquelle (12) zur Erzeugung eines Hornreflexes direkt oder durch eine einfache Glasscheibe (13) auftritt.

**18.** Pupillometer nach einem der Ansprüche 8 bis 17, dadurch gekennzeichnet, daß die Lichtquelle (12) zur Erzeugung eines Hornreflexes eine Vielzahl von elementaren Lichtquellen (16) aufweist, die gemäß einer Transversalen zur allgemeinen Beobachtungsrichtung (D) der Person ausgerichtet sind.

**19.** Pupillometer nach einem der Ansprüche 8 bis 18, dadurch gekennzeichnet, daß es zwei Lichtquellen (12D, 12G) zur Erzeugung eines Hornreflexes gibt, und zwar eine pro Auge.

**20.** Pupillometer nach einem der Ansprüche 8 bis 19, dadurch gekennzeichnet, daß für die Bildung des Meß-Bezugssystem es wenigstens eine Gruppe von Bezugs-Lichtquellen (28) aufweist, die gemäß einem regelmäßigen Abstand längs einer Transversalen zur allgemeinen Beobachtungsrichtung (D) der Person linear angeordnet sind, und die ebenso wie der zu erfassende Hornreflex der Abtastvorrichtung (21) unterworfen bzw. ausgesetzt sind.

**21.** Pupillometer nach Anspruch 20, dadurch gekennzeichnet, daß wenigstens auf einem Teil ihres optischen Weges die den Bezugs-Lichtquellen (28) entsprechenden Lichtstrahlen demselben Pfad folgen wie derjenige, dem die dem (den) zu erfassenden Hornreflex (en) entsprechenden Lichtstrahlen folgen.

**22.** Pupillometer nach Anspruch 21, dadurch gekennzeichnet, daß eine Scheibe (13) vorgesehen ist, über die die Lichtquelle (12) geeignet zur Erzeugung eines Hornreflexes auftritt, wobei die Bezugs-Lichtquellen (28) in der Nachbarschaft der Scheibe (13) angeordnet sind und diese einen Spiegel bildet, der für die Lichtstrahlen, die ihn treffen, reflektierend ist.

**23.** Pupillometer nach einem der Ansprüche 20 bis 22, dadurch gekennzeichnet, daß die Gruppe der Bezugs-Lichtquellen (28) durch ein Gitter von parallelen zylindrischen Mikrolinsen gebildet ist, hinter dem wenigstens eine richtige Lichtquelle (29) angeordnet ist.

**24.** Pupillometer nach einem der Ansprüche 20 bis 23, dadurch gekennzeichnet, daß es zwei Gruppen (27D, 27G) von Bezugs-Lichtquellen (28) gibt, und zwar eine pro Auge.

**25.** Pupillometer nach einem der Ansprüche 8 bis 24, dadurch gekennzeichnet, daß der Erfassungsempfänger (20) ein fotoempfindlicher Empfänger ist.

## FIG.1

## FIG.2

## FIG.8A

## FIG.8B

FIG.3

FIG.5

FIG.4

FIG.6

FIG.7